Europäisches Patentamt

European Patent Office

Office européen des brevets

(11) Publication number: **0 336 412**

**A2**

(12) **EUROPEAN PATENT APPLICATION**

(21) Application number: **89106059.2**

(51) Int. Cl.⁴: **C12Q 1/68**

(22) Date of filing: **06.04.89**

(30) Priority: **08.04.88 IT 2013288**

(43) Date of publication of application:
**11.10.89 Bulletin 89/41**

(84) Designated Contracting States:
**AT BE CH DE ES FR GB GR IT LI LU NL SE**

(71) Applicant: **SCLAVO S.p.A.**
**Via Fiorentina 1**
**I-53100 Siena(IT)**

(72) Inventor: **Ratti,Giulio**
**Via Strada di Vico Alto,6**
**I-53100 Siena(IT)**
Inventor: **Comanducci,Maurizio**
**Via Massetana Romana,12/A**
**I-53100 Siena(IT)**
Inventor: **Ricci,Stefano**
**Via Massetana,13**
**I-53100 Siena(IT)**
Inventor: **Garuti,Giancarlo**
**Via C. Prampolini,2/A**
**I-42048 Rubiera (Reggio Emilia)(IT)**
Inventor: **Cosco,Egidio**
**Via Puglie,11**
**I-53100 Siena(IT)**

(74) Representative: **Perani, Aurelio et al**
**c/o JACOBACCI-CASETTA & PERANI 7, Via**
**Visconti di Modrone**
**I-20122 Milano(IT)**

(54) **Synthetic oligonucleotides useful for the determination of Chlamydia trachomatis in a biological sample.**

(57) The nucleotide sequence of the PCHL1 plasmid which is specific to Chlamydia trachomatis (CT) is reported. Synthetic oligonucleotides with sequences homologous or corresponding to a region of at least 10 consecutive nucleotide bases selected from the sequence of the plasmid are particularly useful for the determination of CT in a biological sample.

Figure 1

The present invention relates to a means and a method for the determination of Chlamydia trachomatis (C. trachomatis) in clinical samples. In particular, the present invention concerns new synthetic oligonucleotides useful for the determination of C. trachomatis and a method using the oligonucleotides.

The invention also concerns a diagnostic kit for the detection of C. trachomatis in clinical samples, comprising one or more of the synthetic oligonucleotides.

Chlamydia are Gram-negative bacteria which, like viruses have the characteristic of being obligate intracellular parasites.

For this reason, and due to the fact that they are much smaller than normal bacteria, so as to be invisible by direct microscopic examination, chlamydia have in the past been classified as viruses.

The bacteria have an infective and extracellular form which cannot replicate, called an elementary body, and a replicating intracellular form called the reticular body.

After a period of multiplication by binary fission, the reticular bodies are transformed into elementary bodies which emerge from the host cell and go and infect new cells.

Masses or minicolonies of reticular and elementary bodies within the infected cell constitute the "inclusions" which are visible with an optical microscope.

Chlamydia psittaci and Chlamydia trachomatis, indicated briefly hereunder as CT, are the main human pathogens.

In particular, CT, which is now recognised as one of the most common sexually-transmitted pathogens, is responsible for various types of infection, including trachoma (serovars A to K) and lymphogranuloma venereum (serovars L1, L2 and L3).

Moreover, as a pathogen of the urino-genital system, the bacterium can induce urethritis and cervicitis.

Women with cervical infection which are not diagnosed or are treated inadequately are defined as at risk for a variety of diseases, for example, such as vaginitis, salpingitis and pelvic inflammation.

During birth this may cause pulmonary infections and conjunctivitis in the newborn (Harrison, H.R. et al (1985) A, J. Obstet. Gynecol. 153, 244; Numazaki, K. et al. (1986) J. Clin. Pathol. 39, 84).

For these reasons, a diagnostic method suitable for the routine analysis of CT cells is particularly desirable.

Moreover, a diagnostic test which is quick and sensitive is particularly advantageous for the selection of the correct antibiotic treatment to be used for combatting C. trachomatis infections.

Up to the present, the bacterium has been investigated, in cases of non-gonococcal urethritis and in some cases of cervicitis, by means of culture-isolation, or directly on the sample, by means of poly- or monoclonal antibodies and subsequent microscope examination with fluorescent (immunofluorescence test).

The first method (Schachter J. Chlamydial Infections Engl. J. Med. 78 298, 428, 490 and 540) which comprises the infection of McCoy cells with the sample under test, the suitable culture of the cells and finally a microscopic search for the typical cytoplasmic inclusions, requires specialised personnel and laboratories.

Moreover, the method requires the presence of living chlamydiae in the sample to be analysed, that is chlamydiae which have not been killed by any treatment of the patient with antiseptics or by the method of its removal and transportation.

The presence of factors used to decrease the virulence of the bacterium or particular conditions used to preserve the sample may also interfere with the CT analysis.

As a result, this culture method is lengthy, difficult, and not very suitable for routine application.

However, the immunofluorenscence test, whilst requiring less time for its execution and being simpler is not sufficiently reliable.

New methods have therefore recently been proposed in the art for the determination of C. trachomatis, which are based on techniques for the hybridisation of nucleic acids with marked DNA probes.

In general this technique, which was described for the first time by Nygaard and Hall (Mol. Biol., 9:1250142 (1964) is based on the ability of single-stranded nucleic acids to ligate or hybridise, under certain operative conditions, to a polynucleotide (probe) having a sequence which is homologous to at least one sequence contained in the single strand and is correlated to a certain disease.

The formation of the hybrid (the reconstruction of the double helix) between the probe and the single stranded nucleic acids contained in the sample test indicates that the determinant sequence of the suspected disease is present.

The visual display of the hybrid produced is then carried out with the use of markers.

The diagnostic test has been used for the diagnosis of infectious diseases since it enables the rapid identification of bacteria and viruses.

In investigating infectious microorganisms by means of hybridisation tests with DNA or RNA probes, the target must obviously be a segment of a nucleic acid which is specific to the organism which is to be determined. In the case of bacteria, this may be a particular chromosomal DNA segment, or a plasmid, or may even correspond to a particular ribosomal RNA segment which has been shown to be specific for a particular species or strain. An advantage of the use of the plasmid DNA as the diagnostic target is the fact that multiple copies of these extrachromosomal elements are generally present in the cell which enhances the sensitivity of the test.

A plasmid specific to C. trachomatis has recently been identified and isolated (Lovett, M. et al. (1980) in "Proceedings of 11th ICC and 19th ICAAC Amer. Soc. Microbiol. Washington D.C. 1250-1252).

This plasmid has been used as a diagnostic probe for a hybridisation test in situ in cytology with the use of a radioactive tracer (Palmer, and Falkow, S. (1986) Plasmid 16:52-62; Horn, J.E. et al (1986) J. Infect. Dis. 153:1155). In practice, the probe used is produced by subjecting preparations of the plasmid to enzymatic transcription in vitro in the presence of a nucleotide triphosphate precursor marked with the radioactive isotope $S^{35}$.

The reaction products, which include a population of polynucleotides of variable length, normally from 200 to 1000 nucleotides, with sequences derived from that of the original plasmid, are then used for detecting the presence of CT in suspected samples.

Whilst showing good specificity, the method however has numerous disadvantages which result, on the one hand from the length of time required for carrying it out (4-5 days), on the other hand from the undesired formation of hybrids between the polynucleotides themselves. In fact by the casual transcription of both the plasmid DNA chains, complementary polynucleotides are obtained which can form hybrids with each other.

This hybridisation between the components of the probe itself, which is in competition with the desidered hybridisation between the probe and the target DNA, causes a loss of effectiveness of the diagnostic test.

The object of the present invention is therefore to establish a method which is simple, reproducible and quick, as well as a means, for the determination of Chlamydia trachomatis in a clinical sample.

According to the present invention, this object is achieved by means of methods which operate with new synthetic oligonucleotides which can bind specifically to the plasmid of C. trachomatis. A subject of the present invention is therefore synthetic oligonucleotides useful for the specific determination of Chlamydia trachomatis.

A further subject of the present invention is a method for the determination of Chlamydia trachomatis in clinical samples, which uses one or more of the synthetic oligonucleotides.

Another subject of the present invention is constituted by the use of the synthetic oligonucleotides for the amplification of a DNA fragment of the plasmid specific to Chlamydia trachomatis.

A further subject of the presence invention is a diagnostic kit for the determination of Chlamydia trachomatis in a clinical sample, comprising one or more of the synthetic oligonucleotides.

Further subjects of the present invention will become clear from the following description and examples.

The present invention is based essentially on the determination of the nucleotide sequence of a 7.5 Kb plasmid (PCHL1) specific to C. trachomatis.

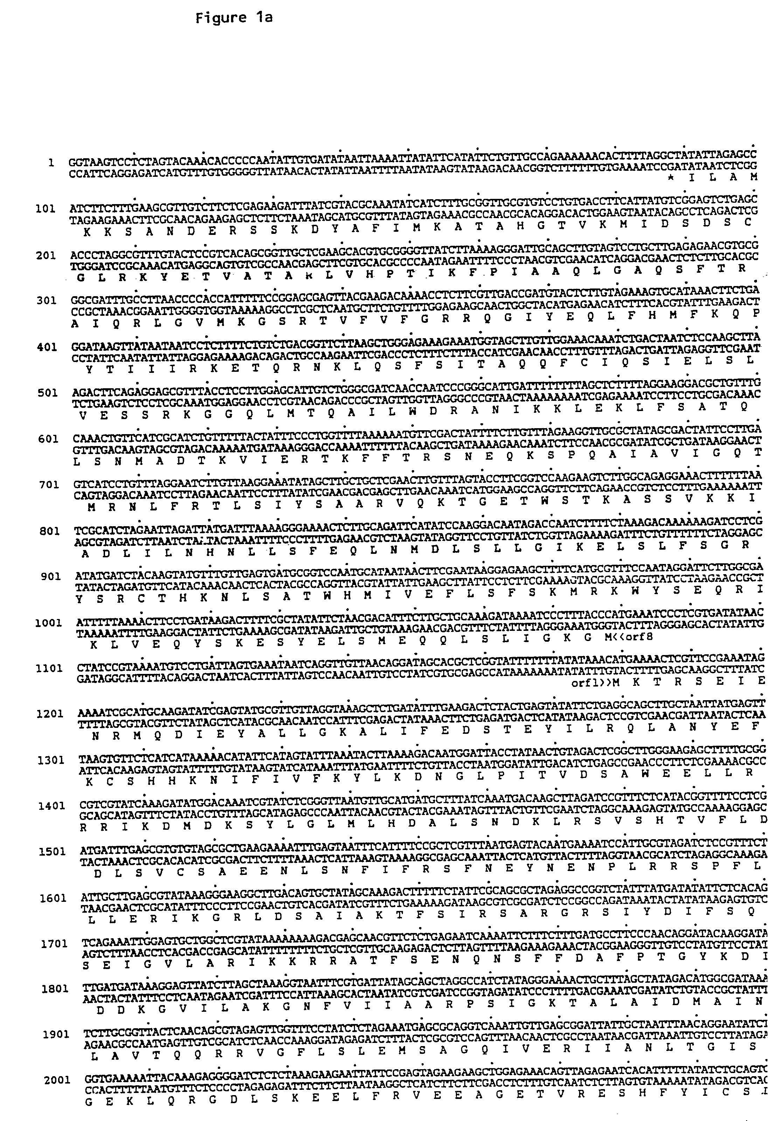

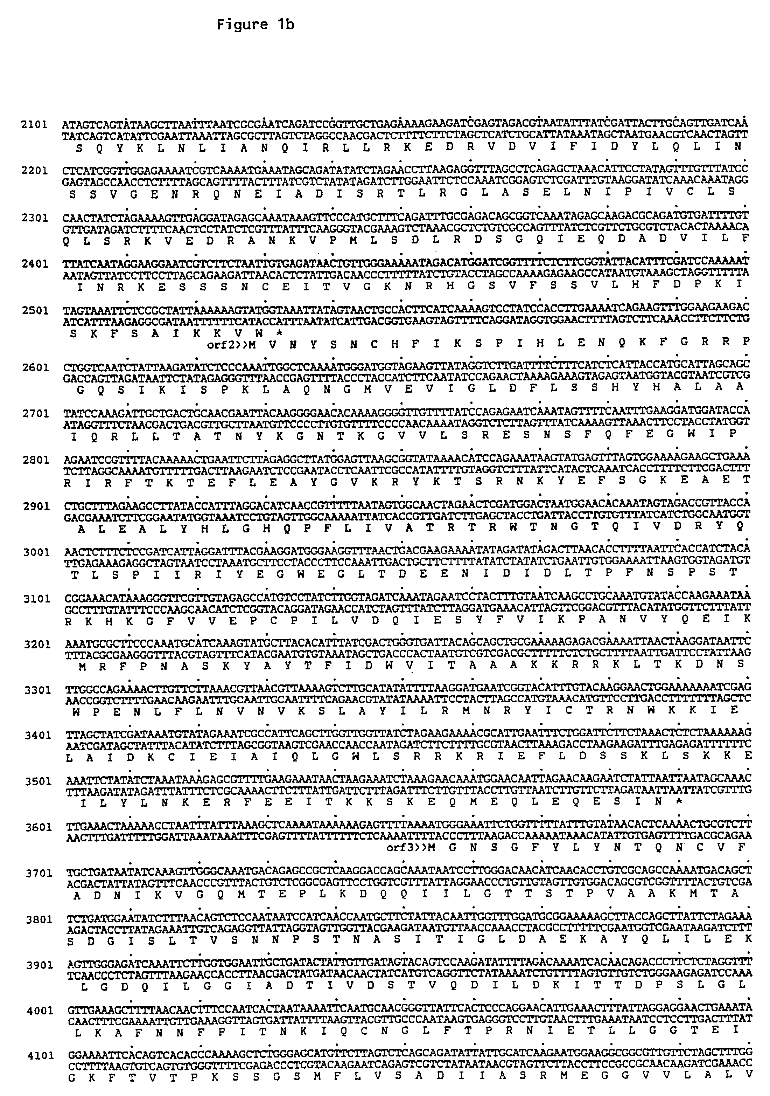

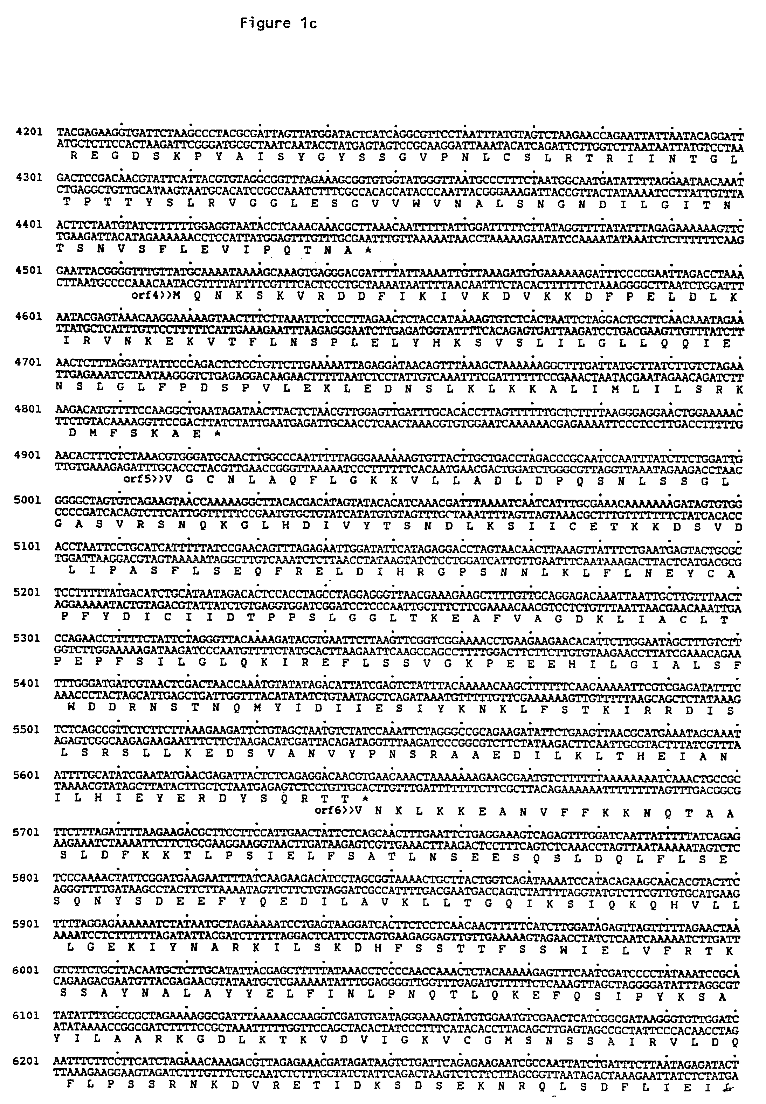

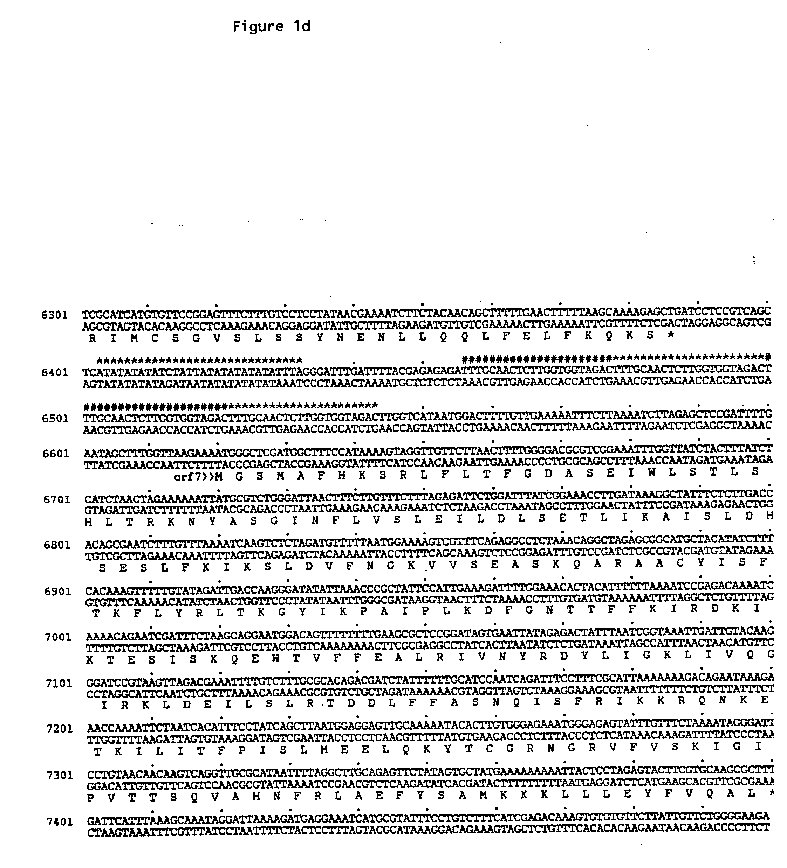

In fact, this enables the synthesis of oligonucleotides with controllable lengths and sequences, which are particularly useful for the detection of Chlamydia trachomatis by means of a hybridisation test, and for the amplication of any PCHL1 plasmid segment which may be present in the sample under test. According to the present invention, said plasmid pCHL1 has been sequenced from the publicly available strain C. trachomatis L2/434/Bn (ATCC VR 902) with the use of generally-known techniques. The sequence, which falls within the scope of the present invention, is constituted by 7449 base pairs (bp) (Figure 1).

The distribution of the bases, which does not differ much in the two DNA helices, is as follows: 33% adenine (A), 31% thymidine (T), 19% guanine (G) and 17% cytosine (C).

Moreover, eight open-reading frames (ORF) were identified in the sequence, of which some seem to contain, in their 5' terminal region, sequences which resemble the promotors of E. coli.

An investigation of the termination signals, carried out according to the method of Brendel and Trifonov, (1984) (Nucl. Acids Res. 12:4411-4427) shows the presence of numerous potential termination sites, some of which are present at the ends of ORF2. ORF3, ORF5 and ORF8.

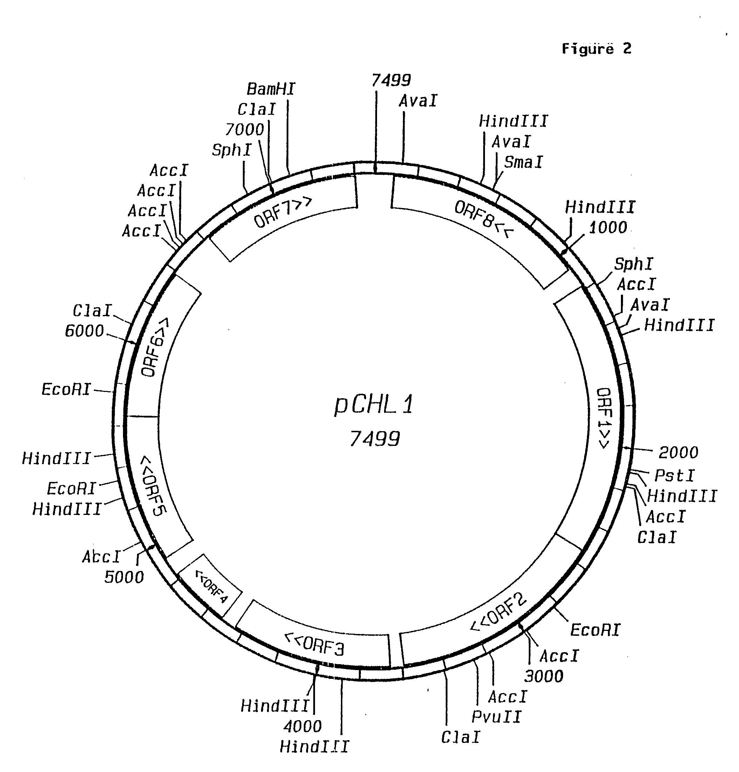

An important characteristic which emerges from the analysis of the sequence is the structure of a region of 200 hp between ORF6 and ORF7 (Figures 1 and 2).

This region comprises a sequence of 30 hp, in which the bases A and T alternate, followed by a sequence of 22 bp which is repeated four times consecutively. This repeated sequence contains the AccI restriction site (GTAGAC), the palindromic sequence TTGCAA and the sequence TGGTGG which is correlated to the Chi octamer which stimulates recombination in E. coli (Smith G.R. et al (1981) Cell, 24:429-435).

The above sequences are typical of the regions comprising the replication origins of bacterial plasmids (Plasmids in Bacteria, Ed. Helsinki, D.R. et al, 1985, Plenum Press, N.Y.).

There is therefore a good probability that the repeated sequence of 22 bp is the attachment site for a regulator protein. In fact, plasmids often have their replication origins repeated sequences with lengths of from 17 to 24 bp, which are involved in controlling the number of copies and incompatibility of the plasmid (Filutowicz, M. et al. (1985) "Plasmids in Bacteria" p.125-140, Plenum Press, N.Y.).

According to the present invention, the sequence and structure data thus determined were used as a source of information for the synthesis of oligonucleotides for use in the detection of CT in a clinical sample.

In particular, oligonucleotides suitable for the purpose of the present invention are characterised by a nucleotide sequence homologous or corresponding to a region of at least 10 consecutive nucleotide bases selected from the sequence of the PCHL1 plasmid shown in Figure 1.

According to the present invention, preferred oligonucleotides have nucleotide sequence homologous or corresponding to a region of the plasmid DNA with from 20 to 100 consecutive bases and in which the region does not contain palindromes which can cause the oligonucleotide to hybridise with itself, and/or comprise an adequate number of guanines and cytosines to provide the hybrids formed with stability.

Particularly preferred are oligonucleotides which have sequences homologous or corresponding to repeated sequences contained in the plasmid DNA. In fact one oligonucleotide with the preferred sequence can hybridies several times with the same plasmid molecule which may be present in a sample, consequently increasing the sensitivity of the test. Also particularly preferred are oligonucleotides having sequences homologous or corresponding to sequences of the 200 bp segment between ORF6 and ORF7.

In fact this segment corresponds to a presumed origin of replication of the plasmid and must therefore be retained in the various isolated species of CT. In order better to illustrate the present invention, without, however, wishing to limit it, the following oligonucleotides were synthesised:

CHL1  5'GGATCCGTAA GTTAGACGAA ATTTTGTCTT TGCGCACAGA 3'

CHL2  5'TTCCTATCAG CTTAATGGAG GAGTTGCAAA AATACACTTG 3'

CHL3  5'GATTCATTTA AAGCAAATAG GATTAAAAGA TGAGGAAATC 3'

CHL4  5'CGCAAATATC ATCTTTGCGG TTGCGTGTCC TGTGACCTTC 3'

CHL5  5'AGGGATTGCA GCTTGTAGTC CTGCTTGAGA GAACGTGCGG 3'

CHL6  5'CATTTTTCCG GAGCGAGTTA CGAAGACAAA ACCTCTTCGT 3'

CHL7  5'AGACTTCAGA GGAGCGTTTA CCTCCTTGGA GCATTGTCTG 3

CHL8  5'GTCATCCTGT TTAGGAATCT TGTTAAGGAA ATATAGCTTG 3

CHL9  5'ATATGATCTA CAAGTATGTT TGTTGAGTGA TGCGGTCCAA 3

CHL10  5'CGTCGTATCA AAGATATGGA CAAATCGTAT CTCGGGTTAA 3'

CHL11  5'ATTGCTTGAG CGTATAAAGG GAAGGCTTGA CAGTGCTATA 3'

CHL12  5'TTGATGATAA AGGAGTTATC TTAGCTAAAG GTAATTTCGT 3'

CHL13  5'ATAGTCAGTA TAAGCTTAAT TTAATCGCGA ATCAGATCCG 3'

CHL14  5'CGGAAACATA AAGGGTTCGT TGTAGAGCCA TGTCCTATCT 3'

CHL15  5'TTGGCCAGAA AACTTGTTCT TAAACGTTAA CGTTAAAAGT 3'

CHL16  5'GGAAAATTCA CAGTCACACC CAAAAGCTCT GGGAGCATGT 3'

CHL17 5'GGGGCTAGTG TCAGAAGTAA CCAAAAAGGC TTACACGACA 3'

CHL18 5'TTTGGGATGA TCGTAACTCG ACTAACCAAA TGTATATAGA 3'

CHL19 5'GTCTTCTGCT TACAATGCTC TTGCATATTA CGAGCTTTTT 3'

CHL20 5'AATAGCTTTG GTTAAGAAAA TGGGCTCGAT GGCTTTCCAT 3'

P1 5'CCAAAGCTAT TCAAAATCGG AGCTCTAAGA 3'

P2 5'TATATTTAGG GATTTGATTT TACGAGAGAG 3'

P3 5'TTTGCAACTC TTGGTGGTAG AC 3

According to the present invention, the oligonucleotides can be synthesised by means of one of the generally-known techniques, with the use of an automatic synthesizer.

Methods which use synthetic oligonucleotides according to the present invention, for the determination of C. trachomatis in a clinical sample, can be carried out by means of any hybridisation test. If the plasmid specific to C. trachomatis is present in the sample under test, it will form a stable hybrid with the oligonucleotides. The hybrid can be displayed by the marking of the oligonucleotides with suitable substances. Markers suitable for the purpose may be selected from the isotopic and non-isotopic elements generally used in diagnostic techniques.

Non-isotopic markers are preferably used and of the latter, biotin, which is easily detectable with an avidin-enzyme system, is preferred. The markers may be introduced into the oligonucleotide by the incorporation of modified nucleotides or after the chemical modification of the oligonucleotide itself.

The determination of the marker is then carried out on the basis of the chemical or physical characteristics of the marker itself. According to the present invention, any hybridisation technique may be used for the determination of C. trachomatis with the use of the oligonucleotides of the present invention.

Conventional techniques which are particularly useful include the fixing of the sample or the probe to a solid substrate (hybridisation on solid phase) or the suspension of the sample and the probes in solution (hybridisation in solution). In any case, the initial stage of the methods consists of the lysing of the cells contained in the sample and the denaturing of the nucleic acids thus released so as to obtain the single strands.

The cell lysis can be carried out with the use of mechanical methods, for example such as physical or chemical or sonication.

The denaturing of the nucleic acids thus extracted is preferably carried out by treatment with alkalis which, if desired, can simultaneously lyse the cells.

Typically, a solid-phase hybridisation method comprises:
- the fixing of the single-strand nucleic acids contained in the sample under test to a solid substrate;
- the placing of the support in contact with one or more marked probes under conditions such as to favour the formation of the hybrid, and finally
- the detection of the hybrid formed.

Solid substrates useful for the purposes of the present invention may be selected from those known in the art.

Examples of the substrates are polymer materials, such as nitrocellulose, nylon and fluorinated polyhydrocarbons or materials having reactive chemical groups, or groups which can be activated.

According to a preferred embodiment of the method of the present invention, the sample under test can

6

be tested by hybridisation in situ.

This method consists of the fixing of the denatured nucleic acids extracted from the bacterial cells contained in the sample under test directly on the microscope slide, in the placing of the slide in contact with a mixture of suitably-marked oligonucleotides and finally, in the determination of the hybrids formed.

The hybridisation is carried out in a solution selected from those known under conditions of ionic concentration and temperature which can be determined experimentally and are such as to enable the formation of specific hybrids.

Typically, the hybridisation takes place in a saline solution of suitable concentration, for example constituted by pH7.5 NaCl and Na-citrate at a temperature from 30° C to 60° C.

Hybridisation in situ advantageously enables the DNA to be kept concentrated locally in the cells contained in the sample under test. According to another embodiment of the method according to the present invention, the detection of CT can be carried out by means of hybridisation on DNA extracted from the sample.

This method consists of the extraction of the nucleic acids from the lysed cells, in the denaturing of the nucleic acids, in the placing of the denatured acids in contact with one or more of the oligonucleotides and finally in the determination of the hybrid formed.

Since the quantity of any target plasmid DNA present in the sample may be too low to be detected by classical techniques for example such as Southern blotting, it is possible to amplify a certain portion of the plasmid in vitro with the use of oligonucleotides of the present invention.

This technique, described by Saiki, R.K. et al. (1985) Science 230, 1350; Scharf, S.J. et al. (1986) Science 233, 1076 can in fact only be used if the nucleotide sequence of the fragment which is to be amplified is known. The sequencing of the plasmid specific to CT according to the present invention therefore makes the use of this known method possible.

In general, the method consists of the synthesis of the oligonucleotides of 20-30 bases with sequences which can ligate to the target DNA in predetermined positions on opposite chains.

A DNA polymerase, added to the reaction mixture, then extends the oligonucleotides (primers) by copying the chains on the DNA to be amplified.

The process can be repeated several times, generally from 20 to 30 times, in the presence of an excess of primers.

The result is a great amplification of the DNA fragment between the two primers.

The presence of this amplified fragment can then be detected either by electrophoretic analysis or by hybridisation with a marked probe having a sequence complementary to a sequence of the amplified fragment.

According to the present invention, any fragment selected from the sequence of the plasmid of C. trachomatis can be amplified.

Preferably, a segment of 0.186 kb between ORF6 and ORF7 is amplified.

For this purpose the two oligonucleotides P1 and P2 which ligate to the single-stranded plasmid DNA in positions about 0.185 kb apart and on opposite chains were synthesised and used as primers. The amplification reaction was carried out by the suspension of the DNA under test in an excess of the primers in Taq buffer in the presence of Taq polymerase DNA and the 4 desoxynucleotide triphosphates (dATP, dCTP, dTTP, dGTP).

The mixture thus obtained was kept at 95° C (the denaturing of the nucleic acids), and then at 42° C (hybridisation and transcription).

This method, which constitutes an amplification cycle in vitro, can be repeated several times. According to the present invention, the plasmid DNA thus amplified can be detected either by means of electrophoresis with the use of molecular-weight markers, or by the hybridisation of the fragment with the suitably-marked P3 oligonucleotide.

The oligonucleotides according to the present invention are particularly useful in a diagnostic kit for the determination of C. trachomatis.

A diagnostic kit according to the present invention may contain a mixture of suitably-marked oligonucleotides, a lysis solution, a solid substrate and a detection system specific to the marker used.

Another diagnostic kit according to the present invention may contain two oligonucleotides for the amplification of a DNA fragment of the PCHL1 plasmid, a marked oligonucleotide for the hybridisation of the amplified fragment, a buffer, molecular-weight markers, a DNA polymerase and a system for the detection of the markers.

The methods according to the present invention have considerable advantages in comparison with the state of the art.

Among these can be numbered the high precision of the results and the ease and speed with which they can be carried out. Moreover, the methods can be used in any laboratory, since they require neither specialised instrumentation nor personnel.

## BRIEF DESCRIPTION OF THE DRAWINGS

Figure 1 A, B, C and D give the nucleotide sequence of the PCHL1 plasmid.
In this sequence the eight ORFs and the amino acid sequences of the polypeptides deduced from the nucleotide sequences are indicated. Also shown are: the region between ORF6 and ORF7 which is rich in A - T bases and the repeated sequence of 22 bp.

Figure 2 shows a restriction map of PCHL1. The four Accl sites between ORF6 and ORF7 indicate the positions of the four repeated sequences.

Figure 3 In situ hybridisation on a urethral smear. In A) the presence of C. trachomatis is indicated by the dark precipitates.

B) shows a negative control with a biotinilated DNA probe specific to hepatitis B.

Figure 4a The electrophoretic detection of the 0.186 Kb fragment of the PCHL1 plasmid amplified with the P1 and P2 primers in a sample of the total nucleic acids extracted from CT elementary bodies.

Track 1 = molecular-weight markers

Track 2 = a portion of the reaction mixture after 20 amplification cycles

Track 3 = an equivalent portion of the sample before amplification

Figure 4b An autoradiograph obtained after hybridisation with the 13 oligonucleotide marked with $P^{32}$ on a blot of the gel in Figure 4a.

Track 1 = molecular-weight markers

Track 2 = hybrid between the amplified band and the probe P3.

Track 3 = a quantity of sample equivalent to that present in track 2 but not amplified.

The experiment examples which follow are illustrative of the invention and are not limiting.

## EXAMPLE 1

Determination of the nucleotide sequence of the plasmid specific to C. trachomatis

The strain C. trachomatis L2/434/Bu (ATCC VR 902) was grown on HeLa 229 cells in a static culture in 175 cm³ Flacon flasks at 37°C.

The supporting medium was Eagle's minimal essential medium with the addition of 10% calf fetal serum and the antibiotics Gentalyn and Funginzone.

At the time of innoculation, cycloheximide (Sigma) (1μg/ml) and glucose to a final concentration of 4% were added to the medium.

48 hours after the infection, the elementary bodies were extracted from the infected cells by sonication and then purified on Urografin gradients (Schering) according to the method described by Caldwell et al. (1981). Infect. Immun 31, 1161-1176.

The DNA was then extracted from the elementary bodies and purified by the alkaline lysis method (Maniatis et al). (1982) Molecular cloning: A Laboratory Manual, Cold Spring Harbor).

From a suspension of purified elementary bodies containing approximately 26 mg of protein, approximately 4 mg of total nucleic acids (DNA + RNA) were obtained.

The plasmid DNA was then purified from the total nucleic acids on caesium chloride isopycnic gradients in the presence of ethidium bromide according to the method described by Maniatis et al (1982).

500 ng of the plasmid DNA were then cut with 5 units (U) of BamHI restriction enzyme (Boehringer) in 25μl of 50 mM Tris-HCl solution (pH 7.5), 100 mM NaCl, 10 mM MgCl at 37°C for 1 hour.

The enzyme reaction was stopped at 65°C for 10 minutes.

The plasmid DNA was then ligated with 100 ng of the commercially-available vector pBR322, cut beforehand with BamHI under the conditions described above.

The ligation reaction was carried out in a solution of 20 μl of 66 mM Tris-HCl (pH 7.6), 1 mM ATP, 10 mM MgCl₂, 15 mM Dithiothreitol (DTT) in the presence of 4 U of T4 DNA ligase, at 14°C for 18 hours.

The entire ligation mixture was then used to transform publicly available E. coli HB 101 cells (ATCC 33694) made competent with CaCl₂ and MgCl₂ (Morrison D.A. 1979 - Methods in Enzymology 68, 326-331).

The transformants were then selected on LB agar plates (10g/l bactotryptone, 5g/l bacto-yeast extract, 5g/l NaCl) containing 50 µg/ml of ampicillin, at 37°C for one night.

The recombinant plasmids were isolated from the positive colonies by rapid extraction (Birnboim, H.C. and Dolly, J. (1979) Nucleic Acid Res., 7, 1513) and analysed by electrophoresis to ascertain the presence of the desired plasmid. One of the positive colonies was then used for the large-scale preparation of the plasmid by the culture of E. coli.

The plasmid was purified on caesium chloride gradients and a portion was digested with BamHI under the conditions described above. After the enzyme had been stopped the digestion mixture was loaded onto 0.8% agarose gel and after running at 100 volts for 3 hours, the BamHI - BamHI fragment of approximately 7.5 kilobases (Kb) was electroeluted (Maniatis et al. 1982).

B) Cloning in M13 and sequencing

Portions of the BamHI - BamHI fragment (1 µg) were digested separately with 5 U of each of the following restriction enzymes, HindIII, ClaI, EcoRI (Boehringer) in the buffer and under the conditions suggested by the supplying company. The individual fragments obtained by digestion with HindIII were then cloned in the M13mp11 vector digested with HindIII and in the M13mp8 and M13mp9 vectors, digested with HindIII and BamHI.

The fragments obtained by digestion with ClaI were cloned on M13mp11 digested with AccI and those obtained by digestion with EcoRI in M13mp11 digested with EcoRI.

The ligation reactions were carried out as described above.

The mixtures thus obtained were then used to transform competent JM 101 E. coli cells.

The transformants were selected on LB and top agar with the addition of 0.05 mM of IPTG (isopropyl-B-D-galactopyranoside) and 0.02% X-gal (5-bromo-4-chloro-3-indolyl-D-galactopyranoside) and incubated at 37°C for 12 hours in a thermostatically-controlled chamber.

The recombinant phages present in the white (positive) plaques, were then used for the determination of the sequences of the cloned inserts.

For this purpose, Sanger's method was used (Sanger, F. et al. (1977) P.N.A.S., 74 5463) with the use either of the universal primers of 15 to 17 bases which are complementary to vector sequences, or a series of primers complementary to the sequences of the fragments under test and synthesised on the basis of the sequence data progressively during the course of the work.

In particular, the primers were 20-base oligonucleotides synthesised by an Applied Biosystems 380A automatic synthesizer with the use of phosphor-starch chemistry (Matteucci, M.D.E. and Caruthers, M.H. (1981) J. Am. Chem. Soc. 103, 3185; Beancage, S.L. and Caruthers, MH. (1981) Tetrahedron Lett. 22, 1859).

Figure 1 gives the nucleotide sequence thus obtained, whilst Figure 2 shows a restriction map of the pCHL1 plasmid.

C) Synthesis of oligonucleotides and their marking with biotin

On the basis of the sequence data (Figure 1), 20 oligonucleotides automatic synthesizer, 2 oligonucleotides of 30 bases (P1 and P2) and one of 22 bases (P3) of 40 bases, identified by the symbol CHL, were synthesised by means of an Applied Biosystems 380A and have the following sequences:

CHL1     5'GGATCCGTAA GTTAGACGAA ATTTTGTCTT TGCGCACAGA3'

CHL2     5'TTCCTATCAG CTTAATGGAG GAGTTGCAAA AATACACTTG3'

CHL3     5'GATTCATTTA AAGCAAATAG GATTAAAAGA TGAGGAAATC3'

CHL4     5'CGCAAATATC ATCTTTGCGG TTGCGTGTCC TGTGACCTTC3'

CHL5     5'AGGGATTGCA GCTTGTAGTC CTGCTTGAGA GAACGTGCGG3'

CHL6     5'CATTTTTCCG GAGCGAGTTA CGAAGACAAA ACCTCTTCGT3'

CHL7     5'AGACTTCAGA GGAGCGTTTA CCTCCTTGGA GCATTGTCTG3'

CHL8     5'GTCATCCTGT TTAGGAATCT TGTTAAGGAA ATATAGCTTG3'

CHL9     5'ATATGATCTA CAAGTATGTT TGTTGAGTGA TGCGGTCCAA3'

CHL10     5'CGTCGTATCA AAGATATGGA CAAATCGTAT CTGGGTTAA3'

CHL11     5'ATTGCTTGAG CGTATAAAGG GAAGGCTTGA CAGTGCTATA3'

CHL12     5'TTGATGATAA AGGAGTTATC TTAGCTAAAG GTAATTTCGT3'

CHL13     5'ATAGTCAGTA TAAGCTTAAT TTAATCGCGA ATCAGATCCG3'

CHL14     5'CGGAAACATA AAGGGTTCGT TGTAGAGCCA TGTCCTATCT3'

CHL15     5' TTGGCCAGAA AACTTGTTCT TAAACGTTAA CGTTAAAAGT 3'

CHL16     5' GGAAAATTCA CAGTCACACC CAAAAGCTCT GGGAGCATGT 3'

CHL17     5' GGGGCTAGTG TCAGAAGTAA CCAAAAAGGC TTACACGACA 3'

CHL18     5' TTTGGGATGA TCGTAACTCG ACTAACCAAA TGTATATAGA 3'

CHL19     5' GTCTTCTGCT TACAATGCTC TTGCATATTA CGAGCTTTTT 3'

CHL20     5' AATAGCTTTG GTTAAGAAAA TGGGCTCGAT GGCTTTCCAT 3'

P1     5' CCAAAGCTAT TCAAAATCGG AGCTCTAAGA 3'

P2     5' TATATTTAGG GATTTGATTT TACGAGAGAG 3'

P3     5' TTTGCAACTC TTGGTGGTAG AC 3'

All the oligonucleotides, except P1 and P2 and P3 were marked with biotin at the 5' terminal using Applied Biosystems Inc. (A.B.I.) aminolink - 1 and NHS - biotin (Pierce) according to the preparation and purification techniques suggested by A.B.I.

EXAMPLE 2

Determination of Chlamydia trachomatis by means of in-situ hybridisation

A sample taken from a patient suffering from non-gonoccocal urethritis by means of a urethral swab was smeared onto two microscope slides. The samples were fixed in acetone for 10 minutes, washed twice in 0.5% Triton X-100 at 2 x SSC (SSC = 0.15 M NaCl, 0.015 M sodium citrate, pH 7.5) for 10 minutes and left to dry in air.

One of the two slides was then covered with 10 to 20 µl of hybridisation mixture of 6 x SSC, 4% dextran sulphate, CHL oligonucleotides (1 to 20) at a concentration at 75 µg/ml of total oligonucleotides.

The other slide was treated with the same hybridisation mixture containing biotinilated polynucleotides specific to human hepatitis B virus.

The slides were placed in a container covered with damp filter paper and kept at 90°C in a thermostatically-controlled bath for 5 minutes.

The slides were then kept in an incubator at 42°C for the period of time necessary for the marked probes to hybridise the corresponding DNA fixed to the solid substrate, that is the CT plasmid, if it is present.

In order to eliminate the excess probe, the slides were then washed as follows:

- twice for five minutes each with 0.5 & triton X-100, 6 x SSC at ambient temperature (20-25°C);

- twice for 20 minutes each with 4 x SSC at 42°C;

- twice for 5 minutes with 4 x SSC at ambient temperature.

The detection of the presence of the biotinilated probe in the samples was carried out with a system constituted by a complex of streptavidin and biotinilated alkaline phosphatase (BRL) used according to the instructions of the supplying company.

The enzyme complex is fixed in the sample in correspondence with the biotinilated probe and, in a subsequent development reaction converts the suitable substrate to insoluble coloured compounds which precipitate and colour the sample at the point at which the probe was initially fixed.

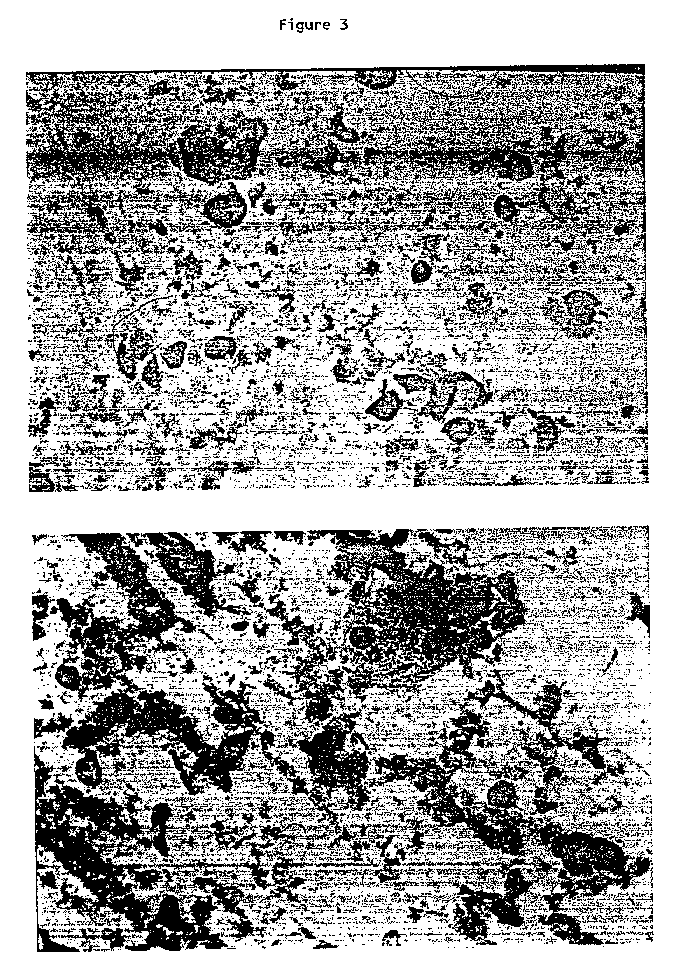

Figure 3 shows the images obtained with an optical microscope (magnified x 40) in which A) shows the cells containing the brown precipitate indicative of the presence of CT and B) shows the similar sample without precipitates, which was tested with the biotinilated probe specific for human hepatitis B.

Both the samples were contrast dyed with eosin.

## EXAMPLE 3

Amplification of the 0.186 Kb plasmid fragment in a sample containing the total nucleic acids of C. trachomatis

500 ng of nucleic acids extracted from the elementary bodies of CT as described in example 1, were suspended in 10 $\mu$l of water.

To this solution were then added
- 10 $\mu$l of Taq 10 x buffer (taq 1x = 16.6 mM ammonium sulphate, 67 mM Tris - HCl, pH 8.8, 6.7 mM MgCl$_2$ 10mM mercaptoethanol) + 170 $\mu$g/ml BSA (bovine serum albumin)
- 2 $\mu$l of an aqueous solution containing 300 picomoles/$\mu$l of each of the primers P1 and P2 - 15 $\mu$l of a 1.25 mM aqueous solution of each of the 4 desoxynucleotide triphosphates
- 62 $\mu$l of water
- 1 $\mu$l of Taq DNA polymerase (Biolabs).

The whole was covered with 100 $\mu$l of mineral oil (Sigma).

The sample was kept at 95° for 3 minutes (denaturing) and then 42°C for 5 minutes (hybridisation and transcription).

The treatment, which constitutes an amplification cycle in vitro was repeated 20 times, the denaturing being carried out for 1 minute in cycles subsequent to the first.

The final result of the amplification was checked as follows:
30 $\mu$l of the mixture were removed and loaded onto a gel constituted by 4% Nusieve agarose (Sigma) and 0.5% ME agarose containing ethidium bromide.

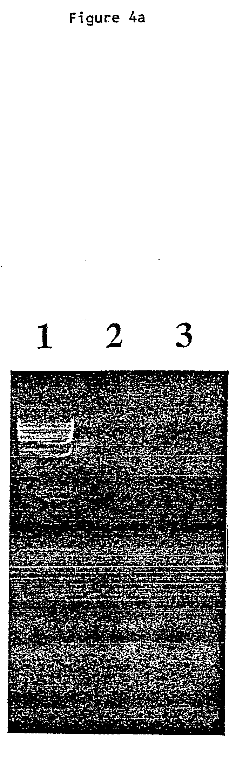

Onto the same gel were loaded a molecular-weight marker, constituted by $\Phi$ x 174 RF DNA digested with HaeIII (Biolabs) (Figures 4A, track 1) and 150 ng of total non-amplified nucleic acids (Figure 4A, track 3).

An electrophoretic band can be seen in track 2 of Figure 4A in correspondence with the 194 bp marker, which indicates strong amplification of the plasmid segment by the two primers.

In order to confirm the results obtained, a Southern blot of gel shown in Figure 4A was hybridised with the P3 oligonucleotide, marked terminally with p$^{32}$ with polynucleotide kinase.

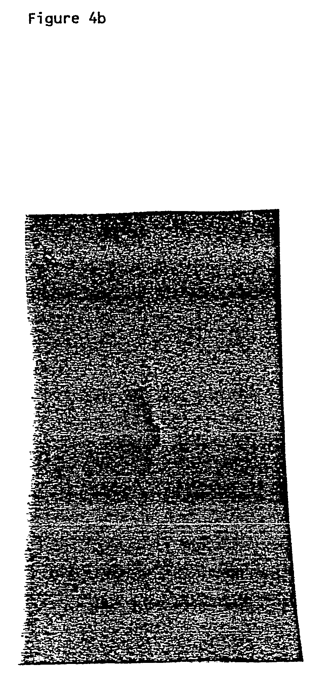

The result shown in Figure 4B shows the specificity of the amplified fragment, confirming the presence of C. trachomatis in the sample analysed.

## EXAMPLE 4

Diagnosis of CT by means of the amplification in vitro of a sample of DNA extracted from human epithelial cells

Human uterine cervical epithelial cells, presumed to be infected by Chlamydia, were take by means of an endocervical swab according to normal gynaecological practice and then diluted and lysed in a solution having the following composition:

| - Tris-HCl pH 7.8 | 10 | mM |
|---|---|---|
| - NaCl | 150 | mM |
| - EDTA | 10 | mM |
| - SDS | 0.6 | % |
| - K proteinase | 100 | $\mu$g/ml |

The reaction was carried out at 37°C for 2 hours.

The total nucleic acids were then extracted with phenol, phenol/chloroform and chloroform/isoamyl alcohol and then treated with RNAse (50 $\mu$g/ml) at 37°C for 1-2 hours and then with pronase (100 $\mu$g/ml) at 37°C for 1 hour.

The reaction mixture was extracted again with phenol/chloroform.

The DNA thus extracted was collected from the aqueous phase by precipitation with ethanol and finally transferred in a final volume of 100 $\mu$l.

Approximately 2 $\mu$g of total DNA were obtained.

The amplification reaction was then carried out on a portion corresponding to approximately 500 ng with the use of the synthetic oligonucleotides P1 and P2 and operating as described in Example 3 above.

The experiment described in Example 3 was carried out in parallel as a control.

Portions of the reaction mixtures equal to 1/10 of the volume, were analysed by electrophoresis on agarose gel in the presence of molecular-weight markers.

After dyeing with ethidium bromide the gel appeared similar to that shown in Figure 4A. The presence of a single electrophoretic band of approximately 200 base pairs and having the same mobility as that produced in the control, indicated the presence of the plasmid specific to C. trachomatis in the epithelial cells of the patient.

## Claims

1. Synthetic oligonucleotides useful for the determination of Chlamydia trachomatis in a biological sample characterised by a nucleotide sequence homologous to a region of at least 10 consecutive nucleotide bases selected from the sequence of the plasmid specific to Chlamydia trachomatis, given in Figure 1.

2. Synthetic oligonucleotides according to claim 1, in which the number of consecutive nucleotide bases is between 20 and 100.

3. Synthetic oligonucleotides according to claim 1, in which the nucleotide sequence does not contain palindromes.

4. Synthetic oligonucleotides according to claim 1, in which the nucleotide sequence is homologous to at least one repeated region contained in the sequence of the plasmid specific to Chlamydia trachomatis.

5. Synthetic oligonucleotides according to claim 4, in which the region is contained in the fragment of approximately 200 base pairs between positions 6426 and 6611 of the sequence of the plasmid Chlamydia trachomatis.

6. A method for the determination of Chlamydia trachomatis in a clinical sample, which comprises the denaturing of the double-stranded nucleic acids extracted from the disrupted cells contained in the sample under test, the placing of the single-stranded nucleic acids thus obtained in contact with one or more probes having nucleotide sequences homologous to at least one sequence contained in the strands and the detection of the hybridised probe, the method being characterised in that the probes are selected from the group of oligonucleotides according to claims 1 to 5.

7. A method according to claim 6, in which the single-stranded nucleic acids are ligated to an insoluble solid substrate and then placed in contact, in a hybridisation mixture, with one or more of the oligonucleotides according to claims 1 to 5, suitably marked.

8. A method according to claim 7, in which the marker substances are selected from the group of radioactive isotopes.

9. A method according to claim 7, in which the marker substances are selected from the group of haptens, antigens, antibodies, biotin, fluorescent substances and chemiluminescent substances.

10. A method according to claim 6, characterised in that the determination of Chlamydia trachomatis is carried out by means of the hybridisation in situ of the samples under test.

11. A method according to claim 6, in which a fragment of the single-stranded DNA of the plasmid Chlamydia trachomatis which may be present in the sample under test is amplified in vitro by means of the use of synthetic oligonucleotides according to claim 1, where the oligonucleotides are characterised by sequences capable of ligating to the fragment of plasmid DNA to be amplified at a predetermined distance and on opposite strands.

12. A method according to claim 11, in which the amplified plasmid DNA fragment contains the presumed origin of replication of the pCHL1 plasmid.

13. A method according to claim 12, in which the plasmid DNA fragment is constituted by 0.186 Kilobases and the oligonucleotides are P1 and P2.

14. A method according to claim 11, in which the amplified plasmid DNA fragment is displayed by electrophoresis.

15. A method according to claim 11, in which the amplified plasmid DNA fragment is displayed by hybridisation with a marked oligonucleotide having a sequence complementary to a sequence contained in the amplified fragment.

16. A method according to claim 15, in which the oligonucleotide is P3.

17. A plasmid specific to Chlamydia trachomatis characterised by the nucleotide sequence of 7499 base pairs given in Figure 1.

18. A diagnostic kit for the determination of Chlamydia trachomatis in a biological sample, characterised in that it contains one or more oligonucleotides according to claims 1 to 5.

19. A diagnostic kit according to claim 18, in which the oligonucleotides are marked.

20. A diagnostic kit according to claim 18, which includes two oligonucleotides useful for the amplification of a fragment contained in the DNA sequence of the plasmid of Chlamydia trachomatis which may be present in the sample under test and a marked oligonucleotide which is complementary to a sequence contained in the amplified fragment.

21. A diagnostic kit according to claim 20, in which the oligonucleotides useful for the amplification are P1 and P2 and the marked oligonucleotide is P3.

## Figure 1a

```
   1   GGTAAGTCCTCTAGTACAAACACCCCCAATATTGTGATATAATTAAAAATTATATTCATATTCTGTTGCCAGAAAAAAACACTTTTAGGCTATATTAGAGCC
       CCATTCAGGAGATCATGTTTGTGGGGGTTATAACACTATATTAATTTTAATATAAGTATAAGACAACGGTCTTTTTTGTGAAAATCCGATATAATCTCGG
                                                                                              *  I  L  A  M

 101   ATCTTCTTTGAAGCGTTGTCTTCTCGAGAAGATTTATCGTACGCAAATATCATCTTTGCGGTTGCGTGTCCTGTGACCTTCATTATGTCGGAGTCTGAGC
       TAGAAGAAACTTCGCAACAGAAGAGCTCTTCTAAATAGCATGCGTTTATAGTAGAAACGCCAACGCACAGGACACTGGAAGTAATACAGCCTCAGACTCG
        K  K  S  A  N  D  E  R  S  S  K  D  Y  A  F  I  M  K  A  T  A  H  G  T  V  K  M  I  D  S  D  S  C

 201   ACCCTAGGCGTTTGTACTCCGTCACAGCGGTTGCTCGAAGCACGTGCGGGGGTTATCTTAAAAGGGATTGCAGCTTGTAGTCCTGCTTGAGAGAACGTGCG
       TGGGATCCGCAAACATGAGGCAGTGTCGCCAACGAGCTTCGTGCACGCCCCAATAGAATTTTCCCTAACGTCGAACATCAGGACGAACTCTCTTGCACGC
        G  L  R  K  Y  E  T  V  A  T  A  R  L  V  H  P  T  I  K  F  P  I  A  A  Q  L  G  A  Q  S  F  T  R

 301   GGCGATTTGCCTTAACCCCACCATTTTTCCGGAGCGAGTTACGAAGACAAAACCTCTTCGTTGACCGATGTACTCTTGTAGAAAGTGCATAAACTTCTGA
       CCGCTAAACGGAATTGGGGTGGTAAAAAGGCCTCGCTCAATGCTTCTGTTTTGGAGAAGCAACTGGCTACATGAGAACATCTTTCACGTATTTGAAGACT
        A  I  Q  R  L  G  V  M  K  G  S  R  T  V  F  V  F  G  R  R  Q  G  I  Y  E  Q  L  F  H  M  F  K  Q  P

 401   GGATAAGTTATAATAATCCTCTTTTCTGTCTGACGGTTCTTAAGCTGGGAGAAAGAAATGGTAGCTTGTTGGAAACAAATCTGACTAATCTCCAAGCTTA
       CCTATTCAATATTATTAGGAGAAAAGACAGACTGCCAAGAATTCGACCCTCTTTCTTTACCATCGAACAACCTTTGTTTAGACTGATTAGAGGTTCGAAT
        Y  T  I  I  I  R  K  E  T  Q  R  N  K  L  Q  S  F  S  I  T  A  Q  Q  F  C  I  Q  S  I  E  L  S  L

 501   AGACTTCAGAGGAGCGTTTACCTCCTTGGAGCATTGTCTGGGCGATCAACCAATCCCGGGCATTGATTTTTTTTAGCTCTTTTAGGAAGGACGCTGTTTG
       TCTGAAGTCTCCTCGCAAATGGAGGAACCTCGTAACAGACCCGCTAGTTGGTTAGGGCCCGTAACTAAAAAAAATCGAGAAAATCCTTCCTGCGACAAAC
        V  E  S  S  R  K  G  G  Q  L  M  T  Q  A  I  L  W  D  R  A  N  I  K  K  L  E  K  L  F  S  A  T  Q

 601   CAAACTGTTCATCGCATCTGTTTTTACTATTTCCCTGGTTTTAAAAAAATGTTCGACTATTTTCTTGTTTAGAAGGTTGCGCTATAGCGACTATTCCTTGA
       GTTTGACAAGTAGCGTAGACAAAAATGATAAAGGGACCAAAATTTTTTACAAGCTGATAAAAGAACAAATCTTCCAACGCGATATCGCTGATAAGGAACT
        L  S  N  M  A  D  T  K  V  I  E  R  T  K  F  F  T  R  S  N  E  Q  K  S  P  Q  A  I  A  V  I  G  Q  T

 701   GTCATCCTGTTTAGGAATCTTGTTAAGGAAATATAGCTTGCTGCTCGAACTTGTTTAGTACCTTCGGTCCAAGAAGTCTTGGCAGAGGAAACTTTTTTAA
       CAGTAGGACAAATCCTTAGAACAATTCCTTTATATCGAACGACGAGCTTGAACAAATCATGGAAGCCAGGTTCTTCAGAACCGTCTCCTTTGAAAAAATT
        M  R  N  L  F  R  T  L  S  I  Y  S  A  A  R  V  Q  K  T  G  E  T  W  S  T  K  A  S  S  V  K  K  I

 801   TCGCATCTAGAATTAGATTATGATTTAAAAGGGAAAACTCTTGCAGATTCATATCCAAGGACAATAGACCAATCTTTTCTAAAGACAAAAAAGATCCTCG
       AGCGTAGATCTTAATCTAATACTAAAATTTTCCCTTTTGAGAACGTCTAAGTATAGGTTCCTGTTATCTGGTTAGAAAAGATTTCTGTTTTTTCTAGGAGC
        A  D  L  I  L  N  H  N  L  L  S  F  E  Q  L  N  M  D  L  S  L  L  G  I  K  E  L  S  L  F  S  G  R

 901   ATATGATCTACAAGTATGTGTTGTTGAGTGATGCGGTCCAATGCATAATAACTTCGAATAAGGAGAAGCTTTTCATGCGTTTCCAATAGGATTCTTGGCGA
       TATACTAGATGTTCATACAAACAACTCACTACGCCAGGTTACGTATTATTGAAGCTTATTCCTCTTCGAAAAGTACGCAAAGGTTATCCTAAGAACCGCT
        Y  S  R  C  T  H  K  N  L  S  A  T  W  H  M  I  V  E  F  L  S  F  S  K  M  R  K  W  Y  S  E  Q  R  I

1001   ATTTTTAAAACTTCCTGATAAGACTTTTCGCTATATTCTAACGACATTTCTTGCTGCAAAGATAAAATCCCTTTACCCATGAAATCCCTCGTGATATAAC
       TAAAAATTTTTGAAGGACTATTCTGAAAAGCGATATAAGATTGCTGTAAAGAACGACGTTTCTATTTTAGGGAAATGGGTACTTTAGGGAGCACTATATTG
        K  L  V  E  Q  Y  S  K  E  S  Y  E  L  S  M  E  Q  Q  L  S  L  I  G  K  G  M<<orf8

1101   CTATCCGTAAAATGTCCTGATTAGTGAAATAATCAGGTTGTTAACAGGATAGCACGCTCGGTATTTTTTTATATAAACATGAAAACTCGTTCCGAAATAG
       GATAGGCATTTTACAGGACTAATCACTTTATTAGTCCAACAATTGTCCTATCGTGCGAGCCATAAAAAAATATATTTGTACTTTTGAGCAAGGCTTTATC
                                                                            orf1>>M  K  T  R  S  E  I  E

1201   AAAATCGCATGCAAGATATCGAGTATGCGTTGTTAGGTAAAGCTCTGATATTTGAAGACTCTACTGAGTATATTCTGAGGCAGCTTGCTAATTATGAGTT
       TTTTAGCGTACGTTCTATAGCTCATACGCAACAATCCATTTCGAGACTATAAACTTCTGAGATGACTCATATAAGACTCCGTCGAACGATTAATACTCAA
        N  R  M  Q  D  I  E  Y  A  L  L  G  K  A  L  I  F  E  D  S  T  E  Y  I  L  R  Q  L  A  N  Y  E  F

1301   TAAGTGTTCTCATCATAAAAACATATTCATAGTATTTAAATACTTAAAAGACAATGGATTACCTATAACTGTAGACTCGGCTTGGGAAGAGCTTTTGCGG
       ATTCACAAGAGTAGTATTTTTGTATAAGTATCATAAATTTATGAATTTTCTGTTACCTAATGGATATTGACATCTGAGCCGAACCCTTCTCGAAAACGCC
        K  C  S  H  H  K  N  I  F  I  V  F  K  Y  L  K  D  N  G  L  P  I  T  V  D  S  A  W  E  E  L  L  R

1401   CGTCGTATCAAAGATATGGACAAATCGTATCTCGGGTTAATGTTGCATGATGCTTTATCAAATGACAAGCTTAGATCCGTTTCTCATACGGTTTTTCCTCG
       GCAGCATAGTTTCTATACCTGTTTAGCATAGAGCCCAATTACAACGTACTACGAAATAGTTTACTGTTCGAATCTAGGCAAAGAGTATGCCAAAGGAGC
        R  R  I  K  D  M  D  K  S  Y  L  G  L  M  L  H  D  A  L  S  N  D  K  L  R  S  V  S  H  T  V  F  L  D

1501   ATGATTTGAGCGTGTGTAGCGCTGAAGAAAAATTTGAGTAATTTCATTTTCCGCTCGTTTAATGAGTACAATGAAAATCCATTGCGTAGATCTCCGTTTCT
       TACTAAACTCGCACACATCGCGACTTCTTTTTAAACTCATTAAAGTAAAAGGCGAGCAAATTACTCATGTTACTTTTAGGTAACGCATCTAGAGGCAAAGA
        D  L  S  V  C  S  A  E  E  N  L  S  N  F  I  F  R  S  F  N  E  Y  N  E  N  P  L  R  R  S  P  F  L

1601   ATTGCTTGAGCGTATAAAGGGAAGGCTTGACAGTGCTATAGCAAAGACTTTTTCTATTCGCAGCGCTAGAGGCCGGTCTATTTATGATATATTCTCACAG
       TAACGAACTCGCATATTTCCCTTCCGAACTGTCACGATATCGTTTCTGAAAAAGATAAGCGTCGCGATCTCCGGCCAGATAAATACTATATAAGAGTGTC
        L  L  E  R  I  K  I  K  G  R  L  D  S  A  I  A  K  T  F  S  I  R  S  A  R  G  R  S  I  Y  D  I  F  S  Q

1701   TCAGAAATTGGAGTGCTGGCTCGTATAAAAAAAAGACGAGCAACGTTCTCTGAGAATCAAAATTCTTTCTTTGATGCCTTCCCAACAGGATACAAGGATA
       AGTCTTTAACCTCACGACCGAGCATATTTTTTTTCTGCTCGTTGCAAGAGACTCTTAGTTTTAAGAAAGAAACTACGGAAGGGTTGTCCTATGTTCCTAT
        S  E  I  G  V  L  A  R  I  K  K  R  R  A  T  F  S  E  N  Q  N  S  F  F  D  A  F  P  T  G  Y  K  D  I

1801   TTGATGATAAAGGAGTTATCTTAGCTAAAGGTAATTTCGTGATTATAGCAGCTAGGCCATCTATAGGGAAAACTGCTTTAGCTATAGACATGGCGATAAA
       AACTACTATTTCCTCAATAGAATCGATTTCATTAAAGCACTAATATCGTCGATCCGGTAGATATCCCTTTTGACGAAATCGATATCTGTACCGCTATTT
        D  D  K  G  V  I  L  A  K  G  N  F  V  I  I  A  A  R  P  S  I  G  K  T  A  L  A  I  D  M  A  I  N

1901   TCTTGCGGTTACTCAACAGCGTAGAGTTGGTTTCCTATCTCTAGAAATGAGCGCAGGTCAAATTGTTGAGCGGATTATTGCTAATTTAACAGGAATATCT
       AGAACGCCAATGAGTTGTCGCATCTCAACCAAAGGATAGAGATCTTTACTCGCGTCCAGTTTAACAACTCGCCTAATAACGATTAAATTGTCCTTATAGA
        L  A  V  T  Q  Q  R  R  V  G  F  L  S  L  E  M  S  A  G  Q  I  V  E  R  I  I  A  N  L  T  G  I  S

2001   GGTGAAAAAATTACAAAGAGGGGATCTCTCTAAAGAAGAATTATTCCGAGTAGAAGAAGCTGGAGAAACAGTTAGAGAATCACATTTTTTATATCTGCAGTC
       CCACTTTTTTAATGTTTCTCCCCTAGAGAGATTTCTTCTTAATAAGGCTCATCTTCTTCGACCTCTCTTGTCAATCTCTTAGTGTAAAAAATATAGACGTCAC
        G  E  K  L  Q  R  G  D  L  S  K  E  E  L  F  R  V  E  E  A  G  E  T  V  R  E  S  H  F  Y  I  C  S  I
```

## Figure 1b

```
2101  ATAGTCAGTATAAGCTTAATTTAATCGCGAATCAGATCCGGTTGCTGAGAAAAGAAGATCGAGTAGACGTAATATTTATCGATTACTTGCAGTTGATCAA
      TATCAGTCATATTCGAATTAAATTAGCGCTTAGTCTAGGCCAACGACTCTTTTCTTCTAGCTCATCTGCATTATAAATAGCTAATGAACGTCAACTAGTT
       S  Q  Y  K  L  N  L  I  A  N  Q  I  R  L  L  R  K  E  D  R  V  D  V  I  F  I  D  Y  L  Q  L  I  N

2201  CTCATCGGTTGGAGAAAATCGTCAAAATGAAATAGCAGATATATCTAGAACCTTAAGAGGTTTAGCCTCAGAGCTAAACATTCCTATAGTTTGTTTATCC
      GAGTAGCCAACCTCTTTTAGCAGTTTTACTTTATCGTCTATATAGATCTTGGAATTCTCCAAATCGGAGTCTCGATTTGTAAGGATATCAAACAAATAGG
       S  S  V  G  E  N  R  Q  N  E  I  A  D  I  S  R  T  L  R  G  L  A  S  E  L  N  I  P  I  V  C  L  S

2301  CAACTATCTAGAAAAGTTGAGGATAGAGCAAATAAAGTTCCCATGCTTTCAGATTTGCGAGACAGCGGTCAAATAGAGCAAGACGCAGATGTGATTTTGT
      GTTGATAGATCTTTTCAACTCCTATCTCGTTTATTTCAAGGGTACGAAAGTCTAAACGCTCTGTCGCCAGTTTATCTCGTTCTGCGTCTACACTAAAACA
       Q  L  S  R  K  V  E  D  R  A  N  K  V  P  M  L  S  D  L  R  D  S  G  Q  I  E  Q  D  A  D  V  I  L  F

2401  TTATCAATAGGAAGGAATCGTCTTCTAATTGTGAGATAACTGTTGGGAAAAATAGACATGGATCGGTTTTCTCTTCGGTATTACATTTCGATCCAAAAAT
      AATAGTTATCCTTCCTTAGCAGAAGATTAACACTCTATTGACAACCCTTTTTATCTGTACCTAGCCAAAAGAGAAGCCATAATGTAAAGCTAGGTTTTTA
       I  N  R  K  E  S  S  S  N  C  E  I  T  V  G  K  N  R  H  G  S  V  F  S  S  V  L  H  F  D  P  K  I

2501  TAGTAAAATTCTCCGCTATTAAAAAAGTATGGTAAATTATAGTAACTGCCACTTCATCAAAAGTCCTATCCACCTTGAAAATCAGAAGTTTGGAAGAAGAC
      ATCATTTAAGAGGCGATAATTTTTTCATACCATTTAATATCATTGACGGTGAAGTAGTTTTCAGGATAGGTGGAACTTTTAGTCTTCAAACCTTCTTCTG
       S  K  F  S  A  I  K  K  V  W  *
                        orf2>>M  V  N  Y  S  N  C  H  F  I  K  S  P  I  H  L  E  N  Q  K  F  G  R  R  P

2601  CTGGTCAATCTATTAAGATATCTCCCAAATTGGCTCAAAATGGGATGGTAGAAGTTATAGGTCTTGATTTTCTTTCATCTCATTACCATGCATTAGCAGC
      GACCAGTTAGATAATTCTATAGAGGGTTTAACCGAGTTTTTACCCTACCATCTTCAATATCCAGAACTAAAAGAAAGTAGAGTAATGGTACGTAATCGTCG
       G  Q  S  I  K  I  S  P  K  L  A  Q  N  G  M  V  E  V  I  G  L  D  F  L  S  S  H  Y  H  A  L  A  A

2701  TATCCAAAGATTGCTGACTGCAACGAATTACAAGGGGAACACAAAAGGGGTTGTTTTATCCAGAGAATCAAATAGTTTTCAATTTGAAGGATGGATACCA
      ATAGGTTTCTAACGACTGACGTTGCTTAATGTTCCCCTTGTGTTTTCCCCAACAAAATAGGTCTCTTAGTTTATCAAAAGTTAAACTTCCTACCTATGGT
       I  Q  R  L  L  T  A  T  N  Y  K  G  N  T  K  G  V  V  L  S  R  E  S  N  S  F  Q  F  E  G  W  I  P

2801  AGAATCCGTTTTTACAAAAACTGAATTCTTAGAGGCTTATGGAGTTAAGCGGTATAAAAACATCCAGAAATAAGTATGAGTTTAGTGGAAAAGAAGCTGAAA
      TCTTAGGCAAAATGTTTTTGACTTAAGAATCTCCGAATACCTCAATTCGCCATATTTTGTAGGTCTTTATTCATACTCAAATCACCTTTTCTTCGACTTT
       R  I  R  F  T  K  T  E  F  L  E  A  Y  G  V  K  R  Y  K  T  S  R  N  K  Y  E  F  S  G  K  E  A  E  T

2901  CTGCTTTAGAAGCCTTATACCATTTAGGACATCAACCGTTTTTAATAGTGGCAACTAGAACTCGATGGACTAATGGAACACAAATAGTAGACCGTTACCA
      GACGAAATCTTCGGAATATGGTAAATCCTGTAGTTGGCAAAAATTATCACCGTTGATCTTGAGCTACCTGATTACCTTGTGTTTATCATCTGGCAATGGT
       A  L  E  A  L  Y  H  L  G  H  Q  P  F  L  I  V  A  T  R  T  R  W  T  N  G  T  Q  I  V  D  R  Y  Q

3001  AACTCTTTCTCCGATCATTAGGATTTACGAAGGATGGGAAGGTTTAACTGACGAAGAAAATATAGATATAGACTTAACACCTTTTAATTCACCATCTACA
      TTGAGAAAGAGGCTAGTAATCCTAAATGCTTCCTACCCTTCCAAATTGACTGCTTCTTTTATATCTATATCTGAATTGTGGAAAATTAAGTGGTAGATGT
       T  L  S  P  I  I  R  I  Y  E  G  W  E  G  L  T  D  E  E  N  I  D  I  D  L  T  P  F  N  S  P  S  T

3101  CGGAAACATAAAGGGTTCGTTGTAGAGCCATGTCCTATCTTGGTAGATCAAATAGAATCCTACTTTGTAATCAAGCCTGCAAATGTATACCAAGAAATAA
      GCCTTTGTATTTCCCAAGCAACATCTCGGTACAGGATAGAACCATCTAGTTTATCTTAGGATGAAACATTAGTTCGGACGTTTACATATGGTTCTTTATT
       R  K  H  K  G  F  V  V  E  P  C  P  I  L  V  D  Q  I  E  S  Y  F  V  I  K  P  A  N  V  Y  Q  E  I  K

3201  AAATGCGCTTCCCAAATGCATCAAAGTATGCTTACACATTTATCGACTGGGTGATTACAGCAGCTGCGAAAAAGAGACGAAAATTAACTAAGGATAATTC
      TTTACGCGAAGGGTTTACGTAGTTTCATACGAATGTGTAAATAGCTGACCCACTAATGTCGTCGACGCTTTTTTCTCTGCTTTTAATTGATTCCTATTAAG
       M  R  F  P  N  A  S  K  Y  A  Y  T  F  I  D  W  V  I  T  A  A  A  K  K  R  R  K  L  T  K  D  N  S

3301  TTGGCCAGAAAAACTTGTTCTTAAACGTTAACGTTAAAGTCTTGCATATATTTTAAGGATGAATCGGTACATTTGTACAAGGAACTGGAAAAAAATCGAG
      AACCGGTCTTTTGAACAAGAATTTGCAATTGCAATTTTCAGAACGTATATAAAATTCCTACTTAGCCATGTAAACATGTTCCTTGACCTTTTTTTAGCTC
       W  P  E  N  L  F  L  N  V  N  V  K  S  L  A  Y  I  L  R  M  N  R  Y  I  C  T  R  N  W  K  K  I  E

3401  TTAGCTATCGATAAATGTATAGAAATCGCCATTCAGCTTGGTTGGTTATCTAGAAGAAAACGCATTGAATTTCTGGATTCTTCTAAACTCTCTAAAAAAG
      AATCGATAGCTATTTACATATCTTTAGCGGTAAGTCGAACCAACCAATAGATCTTCTTTTGCGTAACTTAAAGACCTAAGAAGATTTGAGAGATTTTTTC
       L  A  I  D  K  C  I  E  I  A  I  Q  L  G  W  L  S  R  R  R  K  I  E  F  L  D  S  S  K  L  S  K  K  E

3501  AAATTCTATATCTAAATAAAGAGCGTTTTGAAGAAATAACTAAGAAATCTAAAGAACAAATGGAACAATTAGAACAAGAATCTATTAATTAATAGCAAAC
      TTTAAGATATAGATTTATTTCTCGCAAAACTTCTTTATTGATTCTTTAGATTTCTTGTTTACCTTGTTAATCTTGTTCTTAGATAATTAATTATCGTTTG
       I  L  Y  L  N  K  E  R  F  E  E  I  T  K  K  S  K  E  Q  M  E  Q  L  E  Q  E  S  I  N  *

3601  TTGAAACTAAAAACCTAATTTATTTAAAGCTCAAAATAAAAAAGAGTTTTAAAATGGGAAATTCTGGTTTTTATTTGTATAACACTCAAAACTGCGTCTT
      AACTTTGATTTTTGGATTAAATAAATTTCGAGTTTTATTTTTTCTCAAAATTTTACCCTTTAAGACCAAAAATAAACATATTGTGAGTTTTGACGCAGAA
                                                              orf3>>M  G  N  S  G  F  Y  L  Y  N  T  Q  N  C  V  F

3701  TGCTGATAATATCAAAGTTGGGCAAATGACAGAGCCGCTCAAGGACCAGCAAATAATCCTTGGGACAACATCAACACCTGTCGCAGCCAAAATGACAGCT
      ACGACTATTATAGTTTCAACCCGTTTACTGTCTCGGCGAGTTCCTGGTCGTTTATTAGGAACCCTGTTGTAGTTGTGGACAGCGTCGGTTTTACTGTCGA
       A  D  N  I  K  V  G  Q  M  T  E  P  L  K  D  Q  Q  I  I  L  G  T  T  S  T  P  V  A  A  K  M  T  A

3801  TCTGATGGAATATCTTTAACAGTCTCCAATAATCCATCAACCAATGCTTCTATTACAATTGGTTTGGATGCGGAAAAAGCTTACCAGCTTATTCTAGAAA
      AGACTACCTTATAGAAATTGTCAGAGGTTATTAGGTAGTTGGTTACGAAGATAATGTTAACCAAACCTACGCCTTTTTCGAATGGTCGAATAAGATCTTT
       S  D  G  I  S  L  T  V  S  N  N  P  S  T  N  A  S  I  T  I  G  L  D  A  E  K  A  Y  Q  L  I  L  E  K

3901  AGTTGGGAGATCAAATTCTTGGTGGAATTGCTGATACTATTGTTGATAGTACAGTCCAAGATATTTTAGACAAAATCACAACAGACCCTTCTCTAGGTTT
      TCAACCCTCTAGTTTAAGAACCACCTTAACGACTATGATAACAACTATCATGTCAGGTTCTATAAAATCTGTTTTAGTGTTGTCTGGGAAGAGATCCAAA
       L  G  D  Q  I  L  G  G  I  A  D  T  I  V  D  S  T  V  Q  D  I  L  D  K  I  T  T  D  P  S  L  G  L

4001  GTTGAAAGCTTTTAACAACTTTCCAATCACTAATAAAATTCAATGCAACGGGTTATTCACTCCCAGGAACATTGAAACTTTATTAGGAGGAACTGAAATA
      CAACTTTCGAAAATTGTTGAAAGGTTAGTGATTATTTTAAGTTACGTTGCCCAATAAGTGAGGGTCCTTGTAACTTTGAAATAATCCTCCTTGACTTTAT
       L  K  A  F  N  N  F  P  I  T  N  K  I  Q  C  N  G  L  F  T  P  R  N  I  E  T  L  L  G  G  T  E  I

4101  GGAAAAATTCACAGTCACACCCAAAAGCTCTGGGAGCATGTTCTTAGTCTCAGCAGATATTATTGCATCAAGAATGGAAGGCGGCGTTGTTCTAGCTTTGG
      CCTTTTTAAGTGTCAGTGTGGGTTTTCGAGACCCTCGTACAAGAATCAGAGTCGTCTATAATAACGTAGTTCTTACCTTCCGCCGCAACAAGATCGAAACC
       G  K  F  T  V  T  P  K  S  S  G  S  M  F  L  V  S  A  D  I  I  A  S  R  M  E  G  G  V  V  L  A  L  V
```

## Figure 1c

```
4201  TACGAGAAGGTGATTCTAAGCCCTACGCGATTAGTTATGGATACTCATCAGGCGTTCCTAATTTATGTAGTCTAAGAACCAGAATTATTAATACAGGATT
      ATGCTCTTCCACTAAGATTCGGGATGCGCTAATCAATACCTATGAGTAGTCCGCAAGGATTAAATACATCAGATTCTTGGTCTTAATAATTATGTCCTAA
       R  E  G  D  S  K  P  Y  A  I  S  Y  G  Y  S  S  G  V  P  N  L  C  S  L  R  T  R  I  I  N  T  G  L

4301  GACTCCGACAACGTATTCATTACGTGTAGGCGGTTTAGAAAGCGGTGTGGTATGGGTTAATGCCCTTTCTAATGGCAATGATATTTTAGGAATAACAAAT
      CTGAGGCTGTTGCATAAGTAATGCACATCCGCCAAATCTTTCGCCACACCATACCCAATTACGGGAAAGATTACCGTTACTATAAAATCCTTATTGTTTA
       T  P  T  T  Y  S  L  R  V  G  G  L  E  S  G  V  V  W  V  N  A  L  S  N  G  N  D  I  L  G  I  T  N

4401  ACTTCTAATGTATCTTTTTTTGGAGGTAATACCTCAAACAAACGCTTAAACAATTTTTATTGGATTTTTTCTTATAGGTTTTATATTTAGAGAAAAAAGTTC
      TGAAGATTACATAGAAAAAAACCTCCATTATGGAGTTTGTTTGCGAATTTGTTAAAAATAACCTAAAAAGAATATCCAAAATATAAATCTCTTTTTTTCAAG
       T  S  N  V  S  F  L  E  V  I  P  Q  T  N  A  *

4501  GAATTACGGGGTTTGTTATGCAAAATAAAAGCAAAGTGAGGGACGATTTTATTAAAATTGTTAAAGATGTGAAAAAAGATTTCCCCGAATTAGACCTAAA
      CTTAATGCCCCAAACAATACGTTTTATTTTCGTTTCACTCCCTGCTAAAATAATTTTAACAATTTCTACACTTTTTTTCTAAAGGGGCTTAATCTGGATTT
      orf4>>M  Q  N  K  S  K  V  R  D  D  F  I  K  I  V  K  D  V  K  K  D  F  P  E  L  D  L  K

4601  AATACGAGTAAACAAGGAAAAAGTAACTTTCTTAAATTCTCCCTTAGAACTCTACCATAAAGTGTCTCACTAATTCTAGGACTGCTTCAACAAATAGAA
      TTATGCTCATTTGTTCCTTTTTCATTGAAAGAATTTAAGAGGGAATCTTGAGATGGTATTTCACAGAGTGATTAAGATCCTGACGAAGTTGTTTATCTT
       I  R  V  N  K  E  K  V  T  F  L  N  S  P  L  E  L  Y  H  K  S  V  S  L  I  L  G  L  L  Q  Q  I  E

4701  AACTCTTTAGGATTATTCCCAGACTCTCCTGTTCTTGAAAAATTAGAGGATAACAGTTTAAAGCTAAAAAAGGCTTTGATTATGCTTATCTTGTCTAGAA
      TTGAGAAATCCTAATAAGGGTCTGAGAGGACAAGAACTTTTTAATCTCCTATTGTCAAATTTCGATTTTTTCCGAAACTAATACGAATAGAACAGATCTT
       N  S  L  G  L  F  P  D  S  P  V  L  E  K  L  E  D  N  S  L  K  L  K  K  A  L  I  M  L  I  L  S  R  K

4801  AAGACATGTTTTCCAAGGCTGAATAGATAACTTACTCTAACGTTGGAGTTGATTTGCACACCTTAGTTTTTTGCTCTTTTAAGGGAGGAACTGGAAAAAC
      TTCTGTACAAAAGGTTCCGACTTATCTATTGAATGAGATTGCAACCTCAACTAAACGTGTGGAATCAAAAAAACGAGAAAATTCCCTCCTTGACCTTTTTG
       D  M  F  S  K  A  E  *

4901  AACACTTTCTCTAAACGTGGGATGCAACTTGGCCCAATTTTTAGGGAAAAAAGTGTTACTTGCTGACCTAGACCCGCAATCCAATTTATCTTCTGGATTG
      TTGTGAAAGAGATTTGCACCCTACGTTGAACCGGGTTAAAAATCCCTTTTTTCACAATGAACGACTGGATCTGGGCGTTAGGTTAAATAGAAGACCTAAC
      orf5>>V  G  C  N  L  A  Q  F  L  G  K  K  V  L  L  A  D  L  D  P  Q  S  N  L  S  S  G  L

5001  GGGGCTAGTGTCAGAAGTAACCAAAAAGGCTTACACGACATAGTATACACATCAAACGATTTAAAATCAATCATTTGCGAAACAAAAAAAGATAGTGTGG
      CCCCGATCACAGTCTTCATTGGTTTTTCCGAATGTGCTGTATCATATGTGTAGTTTGCTAAATTTTAGTTAGTAAACGCTTTGTTTTTTTCTATCACACC
       G  A  S  V  R  S  N  Q  K  G  L  H  D  I  V  Y  T  S  N  D  L  K  S  I  I  C  E  T  K  K  D  S  V  D

5101  ACCTAATTCCTGCATCATTTTTTATCCGAACAGTTTAGAGAATTGGATATTCATAGAGGACCTAGTAACAACTTAAAGTTATTTCTGAATGAGTACTGCGC
      TGGATTAAGGACGTAGTAAAAATAGGCTTGTCAAATCTCTTAACCTATAAGTATCTCCTGGATCATTGTTGAATTTCAATAAAGACTTACTCATGACGCG
       L  I  P  A  S  F  L  S  E  Q  F  R  E  L  D  I  H  R  G  P  S  N  N  L  K  L  F  L  N  E  Y  C  A

5201  TCCTTTTTATGACATCTGCATAATAGACACTCCACCTAGCCTAGGAGGGTTAACGAAAGAAGCTTTTGTTGCAGGAGACAAATTAATTGCTTGTTTAACT
      AGGAAAAATACTGTAGACGTATTATCTGTGAGGTGGATCGGATCCTCCCAATTGCTTTCTTCGAAACAACGTCCTCTGTTTAATTAACGAACAAATTGA
       P  F  Y  D  I  C  I  I  D  T  P  P  S  L  G  G  L  T  K  E  A  F  V  A  G  D  K  L  I  A  C  L  T

5301  CCAGAACCTTTTTCTATTCTAGGGTTACAAAAGATACGTGAATTCTTAAGTTCGGTCGGAAAACCTGAAGAAGAACACATTCTTGGAATAGCTTTGTCTT
      GGTCTTGGAAAAAGATAAGATCCCAATGTTTTCTATGCACTTAAGAATTCAAGCCAGCCTTTTGGACTTCTTCTTGTGTAAGAACCTTATCGAAACAGAA
       P  E  P  F  S  I  L  G  L  Q  K  I  R  E  F  L  S  S  V  G  K  P  E  E  E  H  I  L  G  I  A  L  S  F

5401  TTTGGGATGATCGTAACTCGACTAACCAAATGTATATAGACATTATCGAGTCTATTTACAAAAACAAGCTTTTTTTCAACAAAAATTCGTCGAGATATTTC
      AAACCCTACTAGCATTGAGCTGATTGGTTTACATATATCTGTAATAGCTCAGATAAATGTTTTTGTTCGAAAAAAGTTGTTTTTAAGCAGCTCTATAAAG
       W  D  D  R  N  S  T  N  Q  M  Y  I  D  I  I  E  S  I  Y  K  N  K  L  F  S  T  K  I  R  R  D  I  S

5501  TCTCAGCCGTTCTCTTCTTAAAGAAGATTCTGTAGCTAATGTCTATCCAAATTCTAGGGCCGCAGAAGATATTCTGAAGTTAACGCATGAAATAGCAAAT
      AGAGTCGGCAAGAGAAGAATTTCTTCTAAGACATCGATTACAGATAGGTTTAAGATCCCGGCGTCTTCTATAAGACTTCAATTGCGTACTTTATCGTTTA
       L  S  R  S  L  L  K  E  D  S  V  A  N  V  Y  P  N  S  R  A  A  E  D  I  L  K  L  T  H  E  I  A  N

5601  ATTTTGCATATCGAATATGAACGAGATTACTCTCAGAGGACAACGTGAACAAACTAAAAAAAGAAGCGAATGTCTTTTTTTAAAAAAAAATCAAACTGCCGC
      TAAAACGTATAGCTTATACTTGCTCTAATGAGAGTCTCCTGTTGCACTTGTTTGATTTTTTTCTTCGCTTACAGAAAAAATTTTTTTTAGTTTGACGGCG
       I  L  H  I  E  Y  E  R  D  Y  S  Q  R  T  T  *
                                   orf6>>V  N  K  L  K  K  E  A  N  V  F  F  K  K  N  Q  T  A  A

5701  TTCTTTAGATTTTAAGAAGACGCTTCCTTCCATTGAACTATTCTCAGCAACTTTGAATTCTGAGGAAAGTCAGAGTTTGGATCAATTATTTTTTATCAGAG
      AAGAAATCTAAAATTCTTCTGCGAAGGAAGGTAACTTGATAAGAGTCGTTGAAACTTAAGACTCCTTTCAGTCTCAAACCTAGTTAATAAAAAATAGTCTC
       S  L  D  F  K  K  T  L  P  S  I  E  L  F  S  A  T  L  N  S  E  E  S  Q  S  L  D  Q  L  F  L  S  E

5801  TCCCAAAACTATTCGGATGAAGAATTTTATCAAGAAGACATCCTAGCGGTAAAACTGCTTACTGGTCAGATAAAATCCATACAGAAGCAACACGTACTTC
      AGGGTTTTGATAAGCCTACTTCTTAAAAATAGTTCTTCTGTAGGATCGCCATTTTGACGAATGACCAGTCTATTTTAGGTATGTCTTCGTTGTGCATGAAG
       S  Q  N  Y  S  D  E  E  F  Y  Q  E  D  I  L  A  V  K  L  L  T  G  Q  I  K  S  I  Q  K  Q  H  V  L  L

5901  TTTTAGGAGAAAAAATCTATAATGCTAGAAAAATCCTGAGTAAGGATCACTTCTCCTCAACAACTTTTTTCATCTTGGATAGAGTTAGTTTTTTAGAACTAA
      AAAATCCTCTTTTTTAGATATTACGATCTTTTTAGGACTCATTCCTAGTGAAGAGGAGTTGTTGAAAAAGTAGAACCTATCTCAATCAAAAATCTTGATT
       L  G  E  K  I  Y  N  A  R  K  I  L  S  K  D  H  F  S  S  T  T  F  S  S  W  I  E  L  V  F  R  T  K

6001  GTCTTCTGCTTACAATGCTCTTGCATATTACGAGCGTTTTTATAAACCTCCCCAACCAAACTCTACAAAAAGAGTTTCAATCGATCCCCTATAAATCCGCA
      CAGAAGACGAATGTTACGAGAACGTATAATGCTCGCAAAAATATTTGGAGGGGTTGGTTTGAGATGTTTTTCTCAAAGTTAGCTAGGGGATATTTAGGCGT
       S  S  A  Y  N  A  L  A  Y  Y  E  L  F  I  N  L  P  N  Q  T  L  Q  K  E  F  Q  S  I  P  Y  K  S  A

6101  TATATTTTGGCCGCTAGAAAAAGGCGATTTAAAAACCAAGGTCGATGTGATAGGGAAAGTATGTGGAATGTCGAACTCATCGGCGATAAGGGTGTTGGATC
      ATATAAAACCGGCGATCTTTTCCGCTAAATTTTTGGTTCCAGCTACACTATCCCTTTCATACACCTTACAGCTTGAGTAGCCGCTATTCCCACAACCTAG
       Y  I  L  A  A  R  K  G  D  L  K  T  K  V  D  V  I  G  K  V  C  G  M  S  N  S  S  A  I  R  V  L  D  Q

6201  AATTTCTTCCTTCATCTAGAAACAAAGACGTTAGAGAAACGATAGATAAGTCTGATTCAGAGAAGAATCGCCAATTATCTGATTTCTTAATAGAGATACT
      TTAAAGAAGGAAGTAGATCTTTGTTTCTGCAATCTCTTTGCTATCTATTCAGACTAAGTCTCTTCTTAGCGGTTAATAGACTAAAGAATTATCTCTATGA
       F  L  P  S  S  R  N  K  D  V  R  E  T  I  D  K  S  D  S  E  K  N  R  Q  L  S  D  F  L  I  E  I  L
```

Figure 1d

```
6301  TCGCATCATGTGTTCCGGAGTTTCTTTGTCCTCCTATAACGAAAATCTTCTACAACAGCTTTTTGAACTTTTTTAAGCAAAAGAGCTGATCCTCCGTCAGC
      AGCGTAGTACACAAGGCCTCAAAGAAACAGGAGGATATTGCTTTTAGAAGATGTTGTCGAAAAACTTGAAAAATTCGTTTTCTCGACTAGGAGGCAGTCG
       R  I  M  C  S  G  V  S  L  S  S  Y  N  E  N  L  L  Q  Q  L  F  E  L  F  K  Q  K  S  *

          **********************************                         ###########################**********************#
6401  TCATATATATATCTATTATATATATATATTTAGGGATTTGATTTTACGAGAGAGATTTGCAACTCTTGGTGGTAGACTTTGCAACTCTTGGTGGTAGACT
      AGTATATATATAGATAATATATATATATAAATCCCTAAACTAAAATGCTCTCTCTAAACGTTGAGAACCACCATCTGAAACGTTGAGAACCACCATCTGA

          ###########################**********************
6501  TTGCAACTCTTGGTGGTAGACTTTGCAACTCTTGGTGGTAGACTTGGTCATAATGGACTTTTGTTGAAAAATTTCTTAAAATCTTAGAGCTCCGATTTTG
      AACGTTGAGAACCACCATCTGAAACGTTGAGAACCACCATCTGAACCAGTATTACCTGAAAACAACTTTTTTAAAGAATTTTAGAATCTCGAGGCTAAAAC

6601  AATAGCTTTGGTTAAGAAAATGGGCTCGATGGCTTTCCATAAAAGTAGGTTGTTCTTAACTTTTTGGGGACGCGTCGGAAATTTGGTTATCTACTTTATCT
      TTATCGAAACCAATTCTTTTACCCGAGCTACCGAAAGGTATTTTCATCCAACAAGAATTGAAAACCCCTGCGCAGCCTTTAAACCAATAGATGAAATAGA
                        orf7>>M  G  S  M  A  F  H  K  S  R  L  F  L  T  F  G  D  A  S  E  I  W  L  S  T  L  S

6701  CATCTAACTAGAAAAAATTATGCGTCTGGGATTAACTTTCTTGTTTCTTTAGAGATTCTGGATTTATCGGAAACCTTGATAAAGGCTATTTCTCTTGACC
      GTAGATTGATCTTTTTTAATACGCAGACCCTAATTGAAAGAACAAAGAAATCTCTAAGACCTAAATAGCCTTTGGAACTATTTCCGATAAAGAGAACTGG
       H  L  T  R  K  N  Y  A  S  G  I  N  F  L  V  S  L  E  I  L  D  L  S  E  T  L  I  K  A  I  S  L  D  H

6801  ACAGCGAATCTTTGTTTAAAATCAAGTCTCTAGATGTTTTTTAATGGAAAAGTCGTTTCAGAGGCCTCTAAACAGGCTAGAGCGGCATGCTACATATCTTT
      TGTCGCTTAGAAACAAATTTTAGTTCAGAGATCTACAAAAAATTACCTTTTTCAGCAAAGTCTCCGGAGATTTGTCCGATCTCGCCGTACGATGTATAGAAA
       S  E  S  L  F  K  I  K  S  L  D  V  F  N  G  K  V  V  S  E  A  S  K  Q  A  R  A  A  C  Y  I  S  F

6901  CACAAAGTTTTTGTATAGATTGACCAAGGGATATATTAAACCCGCTATTCCATTGAAAGATTTTGGAAACACTACATTTTTTAAAATCCGAGACAAAATC
      GTGTTTCAAAAACATATCTAACTGGTTCCCTATATAATTTGGGCGATAAGGTAACTTTCTAAAACCTTTGTGATGTAAAAAATTTTAGGCTCTGTTTTAG
       T  K  F  L  Y  R  L  T  K  G  Y  I  K  P  A  I  P  L  K  D  F  G  N  T  T  F  F  K  I  R  D  K  I

7001  AAAACAGAATCGATTTCTAAGCAGGAATGGACAGTTTTTTTTTGAAGCGCTCCGGATAGTGAATTATAGAGACTATTTAATCGGTAAATTGATTGTACAAG
      TTTTGTCTTAGCTAAAGATTCGTCCTTACCTGTCAAAAAAAACTTCGCGAGGCCTATCACTTAATATCTCTGATAAATTAGCCATTTAACTAACATGTTC
       K  T  E  S  I  S  K  Q  E  W  T  V  F  F  E  A  L  R  I  V  N  Y  R  D  Y  L  I  G  K  L  I  V  Q  G

7101  GGATCCGTAAGTTAGACGAAATTTTGTCTTTGCGCACAGACGATCTATTTTTTGCATCCAATCAGATTTCCTTTCGCATTAAAAAAAAGACAGAATAAAGA
      CCTAGGCATTCAATCTGCTTTAAAACAGAAACGCGTGTCTGCTAGATAAAAAACGTAGGTTAGTCTAAAGGAAAGCGTAATTTTTTTCTGTCTTATTTCT
        I  R  K  L  D  E  I  L  S  L  R  T  D  D  L  F  F  A  S  N  Q  I  S  F  R  I  K  K  R  Q  N  K  E

7201  AACCAAAATTCTAATCACATTTCCTATCAGCTTAATGGAGGAGTTGCAAAAATACACTTGTGGGAGAAATGGGAGAGTATTTGTTTCTAAAAATAGGGATT
      TTGGTTTTAAGATTAGTGTAAAGGATAGTCGAATTACCTCCTCAACGTTTTTATGTGAACACCCTCTTTACCCTCTCATAAACAAAGATTTTATCCCTAA
       T  K  I  L  I  T  F  P  I  S  L  M  E  E  L  Q  K  Y  T  C  G  R  N  G  R  V  F  V  S  K  I  G  I

7301  CCTGTAACAACAAGTCAGGTTGCGCATAATTTTAGGCTTGCAGAGTTCTATAGTGCTATGAAAAAAAAAATTACTCCTAGAGTACTTCGTGCAAGCGCTTT
      GGACATTGTTGTTCAGTCCAACGCGTATTAAAATCCGAACGTCTCAAGATATCACGATACTTTTTTTTTAATGAGGATCTCATGAAGCACGTTCGCGAAA
       P  V  T  T  S  Q  V  A  H  N  F  R  L  A  E  F  Y  S  A  M  K  K  K  L  L  L  E  Y  F  V  Q  A  L  *

7401  GATTCATTTAAAGCAAATAGGATTAAAAGATGAGGAAATCATGCGTATTTCCTGTCTTTCATCGAGACAAAGTGTGTGTTCTTATTGTTCTGGGGAAGA
      CTAAGTAAATTTCGTTTATCCTAATTTTCTACTCCTTTAGTACGCATAAAGGACAGAAAGTAGCTCTGTTTCACACACAAGAATAACAAGACCCCTTCT
```

EP 0 336 412 A2

Figure 2

Figure 3

Figure 4a

Figure 4b